# EUROPÄISCHE PATENTSCHRIFT

(11) **EP 2 716 216 B1**
(45) Veröffentlichungstag und Bekanntmachung des Hinweises auf die Patenterteilung: **11.02.2015**
(21) Anmeldenummer: 13186668.3
(22) Anmeldetag: 30.09.2013
(51) Int. Cl.: A61B 5/15, A61B 5/151, A61B 17/34

(54) **Stechhilfevorrichtung zur Blutprobenentnahme**
Lancing device for taking blood samples
Dispositif d'aide à la piqûre pour le prélèvement d'échantillons de sang

(30) Priorität: 04.10.2012 DE 102012019401
(43) Veröffentlichungstag der Anmeldung: 09.04.2014
(73) Patentinhaber: Gerresheimer Regensburg GmbH, 93047 Regensburg (DE)
(72) Erfinder: Volkmuth, Julia, 93142 Maxhütte-Haidhof (DE); Eder, Andreas, 93051 Regensburg (DE)
(74) Vertreter: Hannke, Christian

(56) Entgegenhaltungen:
- EP-A1- 0 458 451
- EP-A1- 1 649 814
- EP-A1- 2 050 393
- US-A- 6 045 567
- US-A1- 2010 160 942
- US-A1- 2012 203 259

## Beschreibung

Die Erfindung betrifft eine Stechhilfevorrichtung zur Blutprobenentnahme mit einem Gehäuse, mit einem axial verlagerbaren Lanzettenhalterelement zum Haltern einer Lanzette und mit einem Stechfederelement zum Beschleunigen des axial verlagerbaren Lanzettenhalterelements in Stechrichtung und mit einem Auslöseelement zum Einleiten eines Stechvorgangs, bei dem das axial verlagerbare Lanzettenhalterelement mittels des gespannten Stechfederelements in Stechrichtung beschleunigt wird.

Gattungsgemäße Stechhilfevorrichtungen sind aus dem Stand der Technik bereits gut bekannt. Diese bisher bekannten Stechhilfevorrichtungen haben jedoch den Nachteil, dass sie beim Spannen der Stechfeder, also beim Überführen der diesbezüglichen Stechhilfevorrichtung in einen stechbereiten Betriebszustand, unbeabsichtigt ausgelöst werden können. Hierbei ist die Gefahr einer ungewollten Verletzung des Benutzers relativ hoch.

Es ist Aufgabe der vorliegenden Erfindung, gattungsgemäße Stechhilfevorrichtungen dahingehend weiterzuentwickeln, dass sie selbst für einen ungeübten Benutzer sehr betriebssicher zu handhaben und bedienen sind, so dass die Gefahr einer ungewollten Verletzung des Benutzers so gering wie möglich gehalten werden kann.

Die Aufgabe der Erfindung wird von einer Stechhilfevorrichtung zur Blutprobenentnahme mit einem Gehäuse, mit einem axial verlagerbaren Lanzettenhalterelement zum Haltern einer Lanzette, mit einem Stechfederelement zum Beschleunigen des axial verlagerbaren Lanzettenhalterelements in Stechrichtung und mit einem Auslöseelement zum Einleiten eines Stechvorgangs gelöst, bei dem das axial verlagerbare Lanzettenhalterelement mittels des gespannten Stechfederelements in Stechrichtung beschleunigt wird, wobei die Stechhilfevorrichtung eine durch das Auslöseelement auslösbare Sperreinrichtung zum Sperren eines Stechfederelementespannvorgangs aufweist.

Durch einen derartigen Mechanismus ist es ermöglicht, dass ein Spannen der Stechhilfevorrichtung in einen stechbereiten Betriebszustand hinein bzw. ein Spannen des entsprechenden Stechfederelements unmöglich ist, sollte das Auslöseelemente von einem Benutzer bereits betätigt bzw. gedrückt sein. Hierdurch wird ein unbeabsichtigtes Einleiten des Stechvorgangs konstruktiv außergewöhnlich einfach verhindert, so dass auch die Gefahr einer ungewollten Verletzung des Benutzers erheblich reduziert ist.

Insbesondere kann mit der erfindungsgemäßen Sperreinrichtung eine Verschiebung eines verschieblich gelagerten Spannschlittenteils zum Spannen des Stechfederelements zumindest teilweise gesperrt werden.

Insofern sieht eine bevorzugte Ausführungsvariante vor, dass das Auslöseelement derart in dem Gehäuse der Stechhilfevorrichtung angeordnet ist, dass durch eine in Bezug auf die Stechrichtung radial gerichtete Betätigungsbewegung des Auslöseelements eine Verschiebung eines axial verschieblich gelagerten Spannschlittenteils einer Spanneinheit der Stechhilfevorrichtung in eine vordere Maximalschiebeposition hinein blockiert ist.

Ein enormer Vorteil dieser Lösung liegt im Allgemeinen darin, dass die Stechhilfevorrichtung nicht gespannt werden kann, wenn kurz vor dem Spannen oder während des Spannens des Stechfederelements das Auslöseelement gedrückt wird bzw. ist.

Konstruktiv kann eine derartige Sperreinrichtung vielfältig realisiert werden. Eine sehr einfach bauende und damit favorisierte Ausführungsvariante sieht vor, dass das Auslöseelement wenigstens ein Sperrrippenteil zum zumindest teilweisen Sperren einer axialen Spannbewegung des axial verschieblich gelagerten Spannschlittenelements in Stechrichtung umfasst.

Hierdurch kann die vorliegende Sperreinrichtung oder zumindest Teile hiervon besonders einfach in die Realität umgesetzt werden. Dies hat den vorteilhaften Effekt, dass die Stechhilfevorrichtung trotz zusätzlicher Sperreinrichtung sehr bauteilreduziert hergestellt werden kann.

Darüber hinaus ist es vorteilhaft, wenn das Auslöseelement wenigstens ein Sperrrippenteil zum zumindest teilweisen Sperren einer axialen Spannbewegung des axial verschieblich gelagerten Spannschlittenelements in Stechrichtung umfasst. Hierdurch kann die Sperreinrichtung zumindest teilweise baulich extrem einfach bereitgestellt werden.

Erstreckt das wenigstens eine Sperrrippenteil sich von einem Drücker- bzw. Anzeigekopfteil des Auslöseelements ausgehend bis in eine Längserstreckungsschiebeebene eines axial verschieblich gelagerten Spannschlittenteils hinein, in welcher das axial verschieblich gelagerte Spannschlittenteil zumindest teilweise axial verschieblich angeordnet ist, kann eine Sperrung sehr betriebssicher garantiert werden.

Die Funktionalität kann weiter vereinfacht werden, wenn die Längserstreckungsschiebeebene zwischen der Mittellängsachse und dem Auslöseelement angeordnet ist.

Die Funktionalität der Sperrung des axial verschieblich gelagerten Spannschlittenteils kann weiter verbessert werden, wenn das wenigstens eine Sperrrippenteil eine in Bezug auf die Stechrichtung geneigte Sperrschräge aufweist.

Umfasst zudem das wenigstens eine Anschlagteil eine Anschlagschräge, welche einer Sperrschräge eines Sperrrippenteils des Auslöseelements zugewandt und gegenüberliegend angeordnet ist, kann über diese Geometrie eine vorteilhafte Sperrung erzielt werden.

Des Weiteren ist es vorteilhaft, wenn die Sperreinrichtung wenigstens ein zur Stechrichtung fluchtendes Anschlagteil zum Anschlagen an das wenigstens eine Sperrrippenteil umfasst, wobei das Anschlagteil an einem axial verschieblich gelagerten Spannschlittenteil angeordnet ist. Hierdurch kann das axial verschieblich gelagerte Spannschlittenteil präzise mit dem wenigstens einem Sperrrippenteil wechselwirken.

Eine vorteilhafte Weiterentwicklung der vorliegenden Stechhilfevorrichtung kann dadurch erreicht werden, wenn eine Anzeigeeinrichtung zum Anzeigen des jeweiligen Betriebszustandes der Stechhilfevorrichtung vorgesehen ist, wobei die Anzeigeeinrichtung das Auslöseelement zum Einleiten eines Stechvorgangs umfasst, bei dem das axial verlagerbare Lanzettenhalterelement in Stechrichtung beschleunigt wird. Vorteilhafterweise ist das Auslöseelement in einem stechbereiten Betriebszustand der Stechhilfevorrichtung in einer ausgerückten Anzeigeposition weiter außen über die äußere Gehäuseoberfläche hervorstehend angeordnet als in einem hiervon verschiedenen Betriebszustand der Stechhilfevorrichtung.

Durch das vorliegende Auslöseelement kann einem Benutzer insbesondere ein Spannzustand eines Stechfederelements der Stechhilfevorrichtung auf baulich außerordentlich einfache Weise angezeigt werden.

Und zwar nicht nur optisch durch das im Spannzustand weiter über die Gehäuseoberfläche überstehende Auslöseelement sondern vorteilhafterweise zusätzlich auch haptisch, da dieses weiter überstehende Auslöseelement durch den Benutzer hervorragend ertastbar ist.

Vorteilhafterweise werden auch diesbezüglich mit bereits vorhandenen Bauteilkomponenten der Stechhilfevorrichtung weitere Funktionen realisiert.

Hierdurch kann nicht nur auf eine Vielzahl an zusätzlichen Bauteilen, wie etwa eine Mechanik zum Steuern einer Farbanzeigeeinrichtung oder dergleichen, verzichtet werden, sondern auch die Betriebssicherheit der gesamten Stechhilfevorrichtung wird durch die Möglichkeit des Ertastens des Betriebszustands weiter erhöht. Dies ist insbesondere bei Benutzern mit einer Sehschwäche enorm vorteilhaft.

Dies ist speziell bei wiederverwertbaren Stechhilfevorrichtungen mit auswechselbaren Lanzetten vorteilhaft, da der Benutzer diese Stechhilfevorrichtungen selbst immer wieder händisch stechbereit spannt.

Die Stechrichtung fluchtet hierbei vorzugsweise mit der Längsachse in Längserstreckung der Stechhilfevorrichtung, wodurch der Aufbau baulich einfach gehalten werden kann.

Eine weitere vorteilhafte Ausführungsvariante sieht vor, dass das Auslöseelement bei einem gespannten Stechfederelement in Bezug auf die Stechrichtung radial weiter außen über die äußere Gehäuseoberfläche hervorstehend angeordnet ist als bei einem ungespannten Stechfederelement. Hierdurch kann eine sehr vorteilhafte und damit gute Einhandbedienung erzielt werden.

Eine andere bevorzugte Ausführungsvariante sieht darüber hinaus vor, dass das Auslöseelement derart in dem Gehäuse der Stechhilfevorrichtung angeordnet ist, dass es durch ein axial verschieblich gelagertes Spannschlittenteil einer Spanneinheit der Stechhilfevorrichtung aus dem Gehäuse heraus ausrückbar ist.

Das Auslöseelement ist im Allgemeinen idealerweise in einigen von dem Gehäuse ausgestalteten Führungsnuten und/oder -stegen translatorisch und in Bezug auf die axiale Stechrichtung vorzugsweise radial verschieblich gelagert.

Das Auslöseelement kann konstruktiv einfach im Sinne der Erfindung in die ausgerückte Anzeigeposition hinein bewegt werden, wenn das Auslöseelement ein Ausrückrippenteil zum Einleiten von Ausrückkräften in das Ausrückelement umfasst.

Idealerweise umfasst dieses Ausrückrippenteil sogleich das Sperrrippenteil, so dass die vorliegende Konstruktion nochmals wesentlich vereinfacht werden kann.

Vorzugsweise ist das Ausrückrippenteil identisch mit diesem Sperrrippenteil ausgestaltet, so dass Merkmale bezüglich des Ausrückrippenteils auch auf das vorliegende Sperrrippenteil zutreffen können.

Die Stechhilfevorrichtung kann hinsichtlich der vorstehenden Funktionen noch kompakter gebaut werden, wenn auch das Ausrückrippenteil sich von einem Drücker- bzw. Anzeigekopfteil des Auslöseelements ausgehend bis in eine Längserstreckungsschiebeebene eines axial verschieblich gelagerten Spannschlittenteils hinein erstreckt, in welcher das axial verschieblich gelagerte Spannschlittenteil zumindest teilweise axial verschieblich angeordnet ist.

Auch diese Funktionalität kann weiter vereinfacht werden, wenn die Längserstreckungsschiebeebene zwischen der Mittellängsachse und dem Auslöseelement angeordnet ist.

Die Betriebssicherheit der Stechhilfevorrichtung kann weiter erhöht werden, wenn das Ausrückrippenteil eine in Bezug auf die Stechrichtung geneigte Ausrückschräge aufweist.

Ist diese geneigte Ausrückschräge dem axial verschieblich gelagerten Spannschiebeschlittenteil zugewandt, kann die Gefahr verringert werden, dass ein Spannvorgang zu hohe Schiebekräfte erfordert.

Idealerweise sind zwei Ausrückrippenteile vorgesehen, so dass in Querrichtung zur Stechrichtung zwei voneinander beabstandete in Bezug auf die Stechrichtung geneigte Ausrückschrägen vorhanden sind, wodurch die Gefahr eines Verkantens des Auslöseelements etwa in den Führungsnuten- und/oder -stegen des Gehäuses oder dergleichen wesentlich verringert werden kann.

Zum vorteilhaften und sicheren Spannen insbesondere eines Stechfederelements einer Antriebseinheit zum Beschleunigen des axial verlagerbaren Lanzettenhalterelements der Stechhilfevorrichtung umfasst letztere idealerweise eine Spanneinheit mit einem händisch betätigbaren Spannschiebeelement.

Es versteht sich, dass auch die Antriebseinheit vielfältig ausgestaltet sein kann. Vorteilhafterweise weist sie wenigstens zwei Federelemente umfassend das Stechfederelement zum Beschleunigen des axial verlagerbaren Lanzettenhalterelements in die Stechrichtung und ein Rückholfederelement zum Beschleunigen des axial verlagerbaren Lanzettenhalterelements entgegen der Stechrichtung auf. Insbesondere ist hierdurch ein Überschwingen der Federelemente vorteilhafterweise nicht erforderlich, da eine Vorwärtsbewegung während des Stechvorgangs und die sich daran anschließende Rückwärtsbewegung nach dem eigentlichen Stechvorgang durch zwei separate Federelemente realisiert werden. Die hier verwendeten Federelemente umfassen vorzugsweise Schraubenfederelemente.

Entlang der Stechrichtung bewegt sich vorliegend somit zumindest das axial verlagerbare Lanzettenhalterelement translatorisch, um einen Stechvorgang durchzuführen. Es bewegt sich entgegen dieser Stechrichtung translatorisch, um die Lanzette wieder zurück in das Gehäuse zu verlagern.

Mithilfe dieses oben erwähnten Spannschiebeelements kann wenigstens das axial verschieblich gelagerte Spannschlittenteil zum Spannen des Stechfederelements translatorisch in Stechrichtung bewegt werden.

Insofern ist es hinsichtlich einer konstruktiv noch einfacher bauenden und einer sehr leicht handbaren Stechhilfevorrichtung vorteilhaft, wenn die Spanneinheit mit mindestens dem axial verschieblich gelagerten Spannschlittenteil ausgerüstet ist, wobei das axial verschieblich gelagerte Spannschlittenteil wenigstens ein Betätigungselement zum Ausrücken des Auslöseelements umfasst.

Es versteht sich, dass dieses Betätigungselement vielfältig ausgestaltet sein kann. Vorteilhafterweise weist das wenigstens eine Betätigungselement ein in Stechrichtung fluchtendes Armteil auf, welches eine in Bezug auf die Stechrichtung geneigte Betätigungsschräge umfasst, um beim Spannen des Stechfederelements mit dem Ausrückrippenteil derart wechselwirken zu können, dass das Auslöseelement nur mit einem geringen Kräfteaufwand in die ausgerückte Anzeigeposition bewegt wird.

Hierbei ist es vorteilhaft, wenn die geneigte Betätigungsschräge insbesondere der geneigten Ausrückschräge zugewandt ist, da hierdurch eine Wechselwirkung zwischen dem axial verschieblich gelagerten Spannschiebeschlittenteil und dem Auslöseelement wesentlich verbessert werden kann.

Zeichnet sich das Auslöseelement durch zwei Ausrückrippenteile aus, ist es vorteilhaft, wenn das Betätigungsteil alternativ zwei in Stechrichtung und quer hierzu beabstandet angeordnete solcher fluchtende Armelemente aufweist.

Durch die vorstehend beschriebenen Maßnahmen gelingt es einerseits auf baulich und andererseits auf funktionell sehr einfache Weise, dass durch einen simplen Spannvorgang des Stechfederelements hinsichtlich eines translatorischen Schiebens des Spannschiebeelements in Richtung der Längsachse der Stechhilfevorrichtung sogleich, dass das Auslöseelement in die ausgerückte Anzeigeposition radial zur Längsachse ausgerückt wird.

Um das Auslöseelement in dieser ausgerückten Anzeigeposition auch dann noch halten zu können, wenn das Spannschiebeelement durch Federkraft bereits wieder zurückverlagert wurde, zeichnet sich die vorliegende Stechhilfevorrichtung des Weiteren vorteilhafterweise noch durch eine Halteeinrichtung zum temporären Halten des Auslöseelements in dieser ausgerückten Anzeigeposition aus.

Es versteht sich, dass auch diese Halteeinrichtung auf unterschiedlichste Weise konstruktiv realisiert sein kann.

Vorzugsweise umfasst die Halteeinrichtung am Auslöseelement Schnappelemente und am Gehäuse entsprechende von diesen Schnappelementen hinterschnappbare Erhebungselemente.

Hierdurch kann ein sicheres Einschnappen des Auslöseelements in die ausgerückte Anzeigeposition gewährleistet werden. Zudem kann hierdurch ebenfalls baulich einfach sichergestellt werden, dass bis zum händischen Drücken des Auslöseelements dieses sicher in der ausgerückten Anzeigeposition verbleibt.

Sollte die Spanneinheit auch einen in einer Spannposition axial festlegbaren Spannschlittenteil zum Halten des Stechfederelements in einem gespannten Zustand umfassen, ist es vorteilhaft, wenn das festlegbare Spannschlittenteil in dieser Spannposition mittels einer an dem axial verlagerbaren Lanzettenhalterelement einrastbaren Rasteinrichtung temporär axial festgelegt ist, da sich hierdurch der bauliche Aufwand weiter reduzieren und darüber hinaus die Handhabung der Stechhilfevorrichtung weiter vereinfachen lässt.

Konstruktiv vorteilhaft kann auch das festlegbare Spannschlittenteil zumindest teilweise derart in dem Gehäuse gelagert sein, dass es sich ebenfalls entlang der Verlagerungsachse bewegen kann, um in die Spannposition hinein oder aus dieser heraus verlagert werden zu können.

Insofern formuliert diese Stechrichtung auch die Verlagerungsachse entlang welcher bzw. in deren Richtung sich insbesondere das axial verlagerbare Lanzettenhalterelement innerhalb des Gehäuses translatorisch bewegt.

Somit gestaltet idealerweise das axial verlagerbare Lanzettenhalterelement konstruktiv einfach ein Stechschlittenteil innerhalb des Gehäuses der vorliegenden Stechhilfevorrichtung aus.

Eine vorteilhafte Ausführungsvariante sieht vor, dass eine Rasteinrichtung zum axialen Festlegen des festlegbaren Spannschlittenteils ein in Bezug zur Stechrichtung radial auslenkbares Rastarmelement umfasst, welches während des Festlegens des festlegbaren Spannschlittenteils in der Spannposition mit dem axial verlagerbaren Lanzettenhalterelement wechselwirkt. Hierdurch kann die einrastbare Rasteinrichtung baulich besonders einfach umgesetzt werden.

Ein derartiges auslenkbares Rastarmelement kann konstruktiv einfach idealerweise elastisch gebogen werden.

Vorzugsweise ist das elastisch auslenkbare Rastarmelement mindestens in zwei voneinander verschiedenen Wegen elastisch biegbar. Vorzugsweise verlaufen die zwei voneinander verschieden Wege hierbei quer zueinander.

Insofern ist das elastisch auslenkbare Rastarmelement mehrfach biegbar ausgestaltet.

Somit zeichnet sich die Stechhilfevorrichtung idealerweise durch eine Spanneinrichtung mit einem in einer Spannposition, in welcher das Stechfederelement gespannt ist, festlegbaren Spannschlittenteil aus, bei welcher das festlegbare Spannschlittenteil in der Spannposition mittels der Rasteinrichtung der Stechhilfevorrichtung temporär festlegbar ist, wobei die Rasteinrichtung ein mittels des Lanzettenhalterelements rastbares Rastarmelement aufweist, mittels welchem das rastbare Spannschlittenteil in der Spannposition temporär axial festgelegt angeordnet ist, solange das rastbare Rastarmelement an diesem Lanzettenhalterelement festgelegt ist.

Eine weiter bevorzugte Ausführungsvariante sieht des Weiteren vor, dass die Rasteinrichtung derart in dem Gehäuse angeordnet ist, dass ein in Bezug zur Stechrichtung radial auslenkbares Rastarmelement durch eine translatorische Lageveränderung des axial verlagerbaren Lanzettenhalterelements in Stechrichtung radial ausrückbar angeordnet ist, so dass das festlegbare Spannschlittenteil unter Zuhilfenahme eines Druckfederelements translatorisch aus der Spannposition in eine Entspannposition verlagerbar ist. Hierdurch gelingt es auf baulich einfache Weise, das festlegbare Spannschlittenteil wieder in seine ursprüngliche Ruhelage bzw. Entspannposition zu überführen, um die Stechhilfevorrichtung betriebssicher für einen neuen Stechvorgang, beispielsweise für einen gefahrenlosen Lanzettenwechsel, vorzubereiten.

Darüber hinaus ist es vorteilhaft, wenn die Rasteinrichtung in Abhängigkeit von einer relativen Axiallage des axial verlagerbaren Lanzettenhalterelements gegenüber dem Gehäuse mit dem festlegbaren Spannschlittenteil wechselwirkt. Hierdurch kann ein sehr einfacher Aufbau der Mechanik erzielt werden.

Eine weitere konstruktive Vereinfachung kann erzielt werden, wenn die Rasteinrichtung ein Hülsenteil umfasst, welches das axial verlagerbare Lanzettenhalterelement außen zumindest teilweise radial umgibt.

Ein konstruktiv einfaches Wechselwirken zwischen der einrastbaren Rasteinrichtung und dem axial verlagerbaren Lanzettenhalterelement kann erzielt werden, wenn das axial verlagerbare Lanzettenhalterelement eine von der einrastbaren Rasteinrichtung radial hintergreifbaren Rastrippe aufweist, welche zumindest teilweise axial erstreckend an dem verlagerbaren Lanzettenhalterelement angeordnet ist.

Die radial hintergreifbare Rastrippe ist idealerweise derart an dem axial verlagerbaren Lanzettenhalterelement platziert, dass sie von dem auslenkbaren Rastarmelement der einrastbaren Rasteinrichtung unerreichbar ist, wenn sich das axial verlagerbare Lanzettenhalterelement in seiner Stechendlage oder kurz davor befindet.

Eine weitere vorteilhafte Ausführungsvariante sieht vor, dass die an dem axial verlagerbaren Lanzettenhalterelement einrastbare Rasteinrichtung Mittel zum Festlegen des festlegbaren Spannschlittenteils aufweist. Dies ist insbesondere dann vorteilhaft, wenn die einrastbare Rasteinrichtung nicht unmittelbar dem festlegbaren Spannschlitten zugeordnet ist.

Die Mittel zum Festlegen des festlegbaren Spannschlittenteils können hierbei in vielfältiger Gestalt aufgeführt sein. Beispielsweise umfassen sie Federelemente oder Nutelemente einer Feder-Nut-Verbindung, welche mit den jeweils entsprechenden Gegenelementen am festlegbaren Spannschlittenteil wechselwirken können, wenn sich dieses in der Spannposition befindet.

Insbesondere die Bedienung der Stechhilfevorrichtung kann darüber hinaus erheblich erleichtert werden, wenn die Spanneinheit ein weiteres Spannschlittenteil zum Verlagern des festlegbaren Spannschlittenteils umfasst, wobei das weitere Spannschlittenteil ein Formelement zum radialen Einrücken der an dem axial verlagerbaren Lanzettenhalterelement einrastbaren Rasteinrichtung aufweist.

Dieses weitere Spannschlittenteil umfasst das axial verschieblich gelagerte Spannschlittenteil.

Vorteilhafterweise ist dieses weitere Spannschlittenteil mittels des außerhalb des Gehäuses zugänglichen Spannschiebeelements manuell entlang der Verlagerungsachse in Stechrichtung verschiebbar, so dass ein Verlagern des festlegbaren Spannschlittenteils in die Spannposition hinein händisch konstruktiv einfach realisiert werden kann.

Auch das dem weiteren Spannschlittenteil zugeordnete Formelement kann konstruktiv vielfältig gestaltet sein. Hinsichtlich einer bevorzugten Konstruktionsvariante ist es vorteilhaft, wenn dieses Formelement eine gegenüber der Stechrichtung geneigte Kulissenführungsfläche umfasst. Hierdurch ist es baulich sehr platzsparend möglich, eine axial entlang der Verlagerungsachse gerichtete Bewegung des weiteren Spannschlittenteils in eine radial zur Verlagerungsachse gerichtete Bewegung der rastbaren Rasteinrichtung bzw. des auslenkbaren Rastarmelements umzulenken.

Die vorliegende Stechhilfevorrichtung kann allgemein noch kompakter und damit nochmals besser handhabbar gebaut werden, wenn das axial verlagerbare Lanzettenhalterelement, das festlegbare Spannschlittenteil oder das weitere Spannschlittenteil der Spanneinrichtung jeweils ein Hülsenteil umfassen.

Besonders vorteilhaft ist es hierbei, wenn einzelne der Hülsenteile derart in dem Gehäuse angeordnet sind, dass diese entlang der Verlagerungsachse beispielsweise ineinander geschoben werden können.

Insofern ist es außergewöhnlich vorteilhaft, wenn das axial verlagerbare Lanzettenhalterelement, das festlegbare Spannschlittenteil und/oder das weitere Spannschlittenteil eine gemeinsame Verlagerungsachse aufweisen.

Die Verlagerungsachse fällt vorzugsweise mit der Mittellängsachse zusammen.

Besonders vorteilhaft ist es auch, dass die vorliegende Kinematik der Antriebseinheit hinsichtlich des Spann- und des eigentlichen Stechvorgangs im Wesentlichen durch die zwei Spannschlittenteile der Spanneinheit und durch das Stechschlittenteil, welche zumindest teilweise ineinander verschieblich angeordnet sind, umgesetzt wird.

Eine weitere bauliche Vereinfachung der Stechhilfevorrichtung kann erzielt werden, wenn die Stechhilfevorrichtung ein zweiteiliges Gehäuse mit einem Oberschalenteil und einem Unterschalenteil aufweist, an welchen zumindest das axial verschieblich gelagerte Spannschlittenteil direkt translatorisch geführt ist.

Jedenfalls sind das Auslöseelement und das axial verschieblich gelagerte Spannschlittenteil derart in dem Gehäuse der Stechhilfevorrichtung angeordnet, dass mittels eines betätigten Auslöseelements in Abhängigkeit einer Axiallage des axial verschieblich gelagerten Spannschlittenteils entweder ein Spannvorgang des Stechfederelements gesperrt oder ein Stechvorgang eingeleitet werden kann.

Weitere Vorteile, Ziele und Eigenschaften vorliegender Erfindung werden anhand anliegender Zeichnung und nachfolgender Beschreibung erläutert, in welchen beispielhaft eine erfindungsgemäße Stechhilfevorrichtung zur Blutprobenentnahme dargestellt und beschrieben ist.

In der Zeichnung zeigen:
- Figur 1: schematisch eine Gesamtansicht einer Stechhilfevorrichtung zur Blutprobenentnahme mit einer erfindungsgemäßen Sperreinrichtung;
- Figur 2: schematisch eine perspektivische Modellansicht der Stechhilfevorrichtung aus der Figur 1, jedoch ohne Gehäuse;
- Figur 3: schematisch eine erste Funktionsseitenansicht der Stechhilfevorrichtung aus den Figuren 1 und 2 mit aktiver Sperreinrichtung, bei welcher Anschlagteile eines axial verschieblich gelagerten Spannschlittenteils in Bezug auf die Stechrichtung axial gegen Sperrrippenteile des Auslöseelements anliegen;
- Figur 4: schematisch eine weitere Funktionsseitenansicht der Stechhilfevorrichtung aus den Figuren 1 bis 3, bei welcher die Anschlagteile in Bezug auf die Stechrichtung radial unter den Sperrrippenteilen des Auslöseelements angeordnet sind;
- Figur 5: schematisch eine weitere Gesamtansicht der Stechhilfevorrichtung aus den Figuren 1 bis 4 gemäß dem in der Figur 4 gezeigten Betriebszustand;
- Figur 6: schematisch eine rückwärtige Querschnittsansicht der Stechhilfevorrichtung aus den Figuren 1 bis 5 in Höhe des radial ausgerückten Auslöseelements; und
- Figur 7: schematisch eine nochmalige Gesamtansicht der Stechhilfevorrichtung aus den Figuren 1 bis 6 in einem ausgelösten Stechzustand.

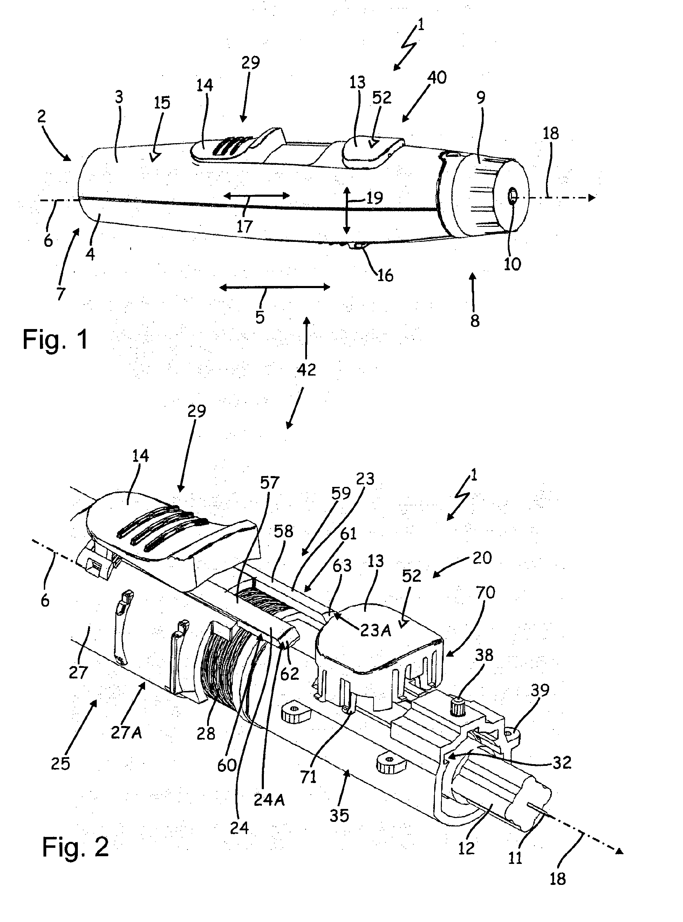

Die in den Figuren 1 bis 7 gezeigte Stechhilfevorrichtung 1 ist für eine Entnahme einer Blutprobe vorgesehen. Die Stechhilfevorrichtung 1 weist ein zweiteiliges Gehäuse 2 mit einem Oberschalenteil 3 und einem Unterschalenteil 4 auf.

Die Stechhilfevorrichtung 1 erstreckt sich mit einer Längserstreckung 5 entlang einer Längsachse 6 von einem Endbereich 7 der Stechhilfevorrichtung 1 zu einem Kopfbereich 8 der Stechhilfevorrichtung 1, wobei in diesem Kopfbereich 8 der Stechhilfevorrichtung 1 eine Schraubkappe 9 mit einer Durchführungsöffnung 10 für eine Stechnadel 11 einer auswechselbaren Lanzette 12 (siehe bspw. Figur 2) der Stechhilfevorrichtung 1 in das zweiteilige Gehäuse 2 eingeschraubt ist. Bei der Längsachse 6 handelt es sich in diesem Ausführungsbeispiel sogleich um die Mittellängsachse der Stechhilfevorrichtung 1.

An dem Oberschalenteil 3 befinden sich radial neben der Längsachse 6 ein Auslösedrückelement 13 zum Einleiten eines Stechvorgangs mit der Stechhilfevorrichtung 1 und ein Spannschiebeelement 14, welche beide über die Mantelfläche 15 des zweiteiligen Gehäuses 2 radial hervorstehen, so dass sie händisch gut erreichbar sind.

An dem Unterschalenteil 4 befindet sich radial neben der Längsachse 6 ein Auswerferschiebeelement 16, welches ebenfalls über die äußere Gehäuseoberfläche 15 des zweiteiligen Gehäuses 2 radial hervorsteht, so dass auch dieses händisch problemlos erreichbar ist.

Während das Spannschiebeelement 14 und das Auswerferschiebeelement 16 in Axialrichtungen 17 und insbesondere in Stechrichtung 18 axial entlang der Längsachse 6 verlagerbar sind, ist das Auslösedrückelement 13 in Radialrichtungen 19 radial in Bezug auf die Längsachse 6 verlagerbar.

Die Darstellung gemäß der Figur 2 zeigt die Stechhilfevorrichtung 1 ohne Gehäuse 2.

Die Stechhilfevorrichtung 1 zeichnet sich durch eine durch das Auslöseelement 13 auslösbare Sperreinrichtung 20 zum Sperren eines Stechfederelementespannvorgangs.

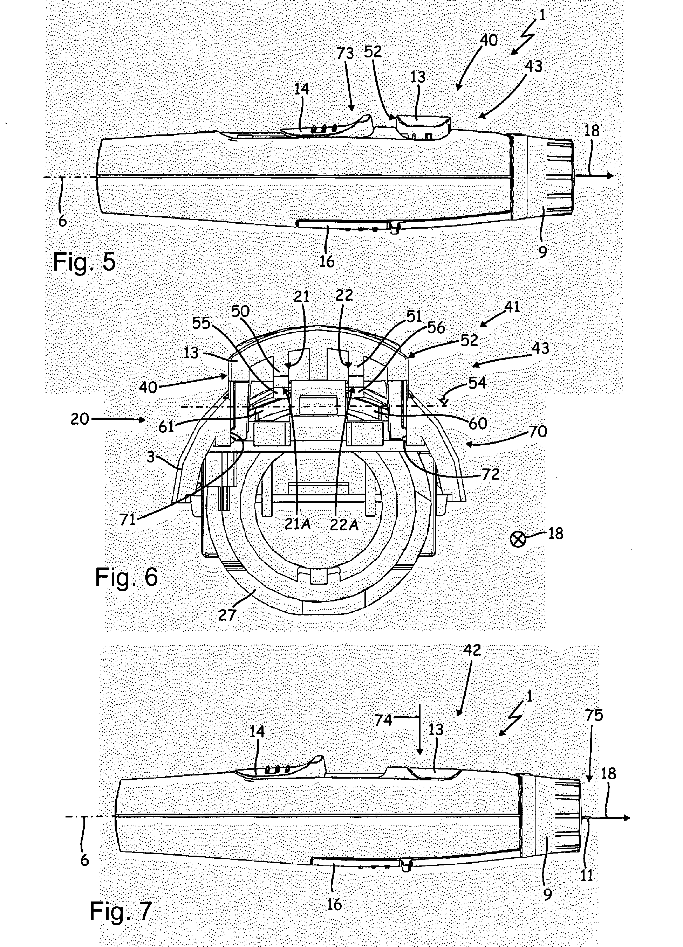

Die Sperreinrichtung 20 zeichnet sich einerseits durch an dem Auslöseelement 13 vorgesehene Sperrrippenteile 21 und 22 aus, welche in Bezug auf die Stechrichtung 18 geneigte Sperrschrägen 21 A und 22A bereitstellen (siehe Figur 6).

Andererseits umfasst die Sperreinrichtung 20 noch zwei zur Stechrichtung 18 fluchtende Anschlagteile 23 und 24 (siehe Figur 2) zum Anschlagen an die Sperrrippenteile 21 und 22, wodurch der Spannvorgang der Stechvorrichtung 1 konstruktiv einfach gesperrt werden kann, wenn das Auslöseelement 13 versehentlich oder beabsichtigt gedrückt ist.

Die Sperrschrägen 21 A und 22A können mit den Anschlagschrägen 23A und 24A beim Sperren des Spannvorgangs vorteilhaft miteinander wechselwirken, da die Sperrschrägen 21A, 22A den Anschlagschrägen 23A und 24A zugewandt und gegenüberliegend angeordnet sind.

Das Spannschiebeelement 14 ist eine Komponente einer Spanneinheit 25 der Stechhilfevorrichtung 1, wobei die Spanneinheit 25 darüber hinaus zumindest ein axial verschieblich gelagertes Spannschlittenteil 27 zum axialen Verlagern eines hier nicht gezeigten festlegbaren Spannschlittenteils sowie ein Spannschlittenfederelement 28 zum Rückverlagern des axial verschieblich gelagerten Spannschlittenteils 27 und des Spannschiebelements 14 in eine hintere Ausgangsposition 29 nach einem Spannvorgang umfasst.

Das festlegbare Spannschlittenteil und das axial verschieblich gelagerte Spannschlittenteil 27 sind nicht fest miteinander fixiert, sondern axial relativ zueinander verschieblich gelagert; jedoch wird das festlegbare Spannschlittenteil von dem axial verschieblich gelagerten Spannschlittenteil 27 mitgeschleppt, wenn das axial verschieblich gelagerte Spannschlittenteil 27 mithilfe des Spannschiebeelements 14 in Stechrichtung 18 händisch verlagert wird.

Sowohl das festlegbare Spannschlittenteil als auch das axial verschieblich gelagerte Spannschlittenteil 27 sind hierbei als Hülsenteile 27A ausgestaltet, wobei das Hülsenteil (hier nicht gezeigt) des festlegbaren Spannschlittenteils zumindest teilweise innerhalb des weiteren Spannschlittenteils 27 axial verschieblich gelagert angeordnet ist, und wobei das Hülsenteil 27A des axial verschieblich gelagerten Spannschlittenteils 27 in den Ober- und Unterschalenteilen 3 und 4 des zweigeteilten Gehäuses 2 axial verschieblich gelagert angeordnet ist.

Das axial verschieblich gelagerte Spannschlittenteil 27 ist mit dem Spannschiebelement 14 formschlüssig verbunden (siehe insbesondere Figur 2), wodurch ersteres mit dem Spannschiebelement 14 problemlos händisch betätigt werden kann.

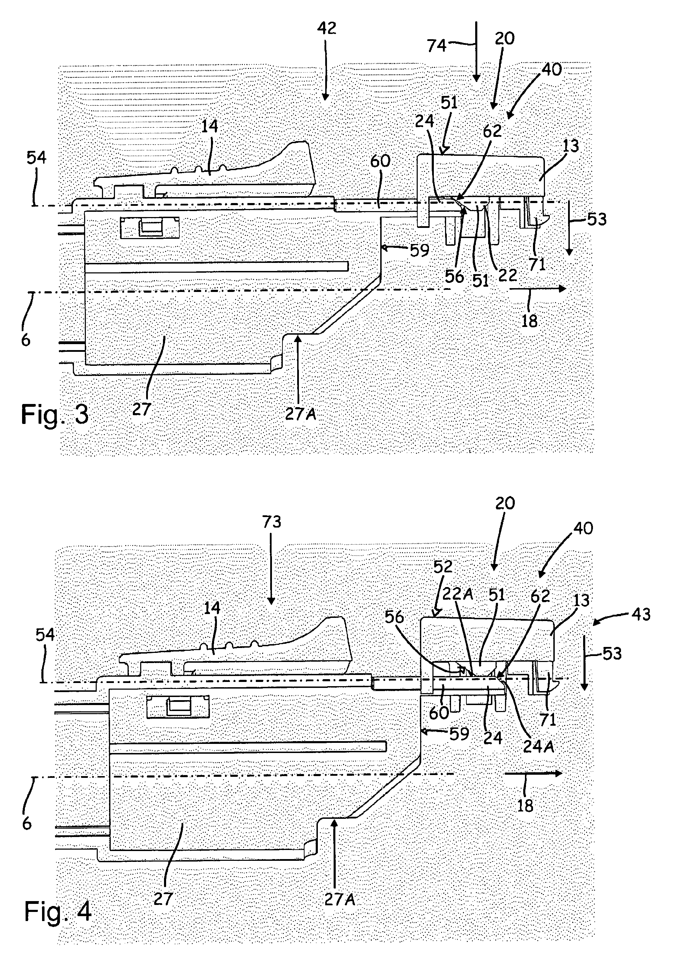

Wird nun dieses Spannschiebelement 14 mit dem axial verschieblich gelagerten Spannschlittenteil 27 in Stechrichtung 18 verschoben, während das Auslöseelement 13 gedrückt ist, kommen die Anschlagschrägen 23A und 24A der an dem axial verschieblich gelagerten Spannschlittenteil 27 angeordneten Anschlagarme 23 und 24 in Kontakt mit den Sperrschrägen 21A, 22A der an dem Auslöseelement 13 vorgesehenen Sperrrippenelemente 21 und 22, wodurch ein weiteres Verlagern insbesondere des axial verschieblich gelagerten Spannschlittenteil 27 in eine vordere Maximalschiebeposition 73 (siehe Figuren 4 und 5) hinein nicht mehr möglich ist. Ein Spannen des Stechfederelements kann nicht durchgeführt werden.

Darüber hinaus umfasst die Stechhilfevorrichtung 1 noch eine Rasteinrichtung (hier nicht gezeigt), welche als Hülsenteil 35 in dem zweiteiligen Gehäuse 2 an dem Ober- und Unterschalenteilen 3 und 4 fest und damit axial verschiebesicher fixiert ist.

Vorteilhafterweise dient das Hülsenteil 35 gleichzeitig auch als Lager- und Führungseinrichtung zum axialen und radialen Lagern eines axial verlagerbaren Lanzettenhalterelements 32, wodurch die Stechhilfevorrichtung 1 nochmals bauteilreduzierter und damit kompakter baut.

Um das Hülsenteil 35 insbesondere verdrehsicher in dem Gehäuse 2 der Stechhilfevorrichtung 1 lagern zu können, weist das Hülsenteil 35 einige Stehbolzen 38 (hier nur exemplarisch beziffert) und Schraubenlaschen 39 (ebenfalls nur exemplarisch beziffert) auf, mithilfe welcher das Hülsenteil 35 an dem Gehäuse 2 ortsfest befestigt werden kann.

Das Auslöseelement 13 gestaltet sogleich eine Anzeigeeinrichtung 40 zum Anzeigen des jeweiligen Betriebszustandes 41 oder 42 der Stechhilfevorrichtung 1 aus.

Befindet sich die Stechhilfevorrichtung 1 in einem stechbereiten Betriebszustand 41 (siehe Figuren 4, 5 und 6), steht das Auslöseelement 13 in einer ausgerückten Anzeigeposition 43 weiter außen über die äußere Gehäuseoberfläche 15 der Stechhilfevorrichtung 1 hervor als in hiervon verschiedenen Betriebszuständen 42 (siehe Figuren 1, 2, 3 und 7), bei welchen die Stechhilfevorrichtung 1 noch nicht stechbereit oder nicht mehr stechbereit ist.

In allen von dem stechbereiten Betriebszustand 41 verschiedenen Betriebszuständen 42 befindet sich das Auslöseelement 13 nicht in der ausgerückten Anzeigeposition 43. Hierdurch wird der stechbereite Betriebszustand 41 vorteilhaft optisch, haptisch und konstruktiv auf extrem einfache Weise signalisiert und hervorgehoben bzw. realisiert.

Insofern ist das Auslöseelement 13 derart in dem Gehäuse 2 angeordnet ist, dass es von dem axial verschieblich gelagerten Spannschlittenteil 27 radial aus dem Gehäuse 2 ausrückbar ist.

Es versteht sich, dass diese Funktionsweise konstruktiv in vielfältiger Weise umgesetzt werden kann.

Bei dem vorliegenden Ausführungsbeispiel verfügt das Auslöseelement 13 über zwei Ausrückrippenteile 50 und 51 (siehe insbesondere Figur 6) zum Einleiten von Ausrückkräften in das Auslöseelement 13 und damit auch zum radialen Ausrücken des Auslöseelements 13 aus dem Gehäuse 2 der Stechhilfevorrichtung 1 heraus in die ausgerückte Anzeigeposition 43 hinein.

Die zwei Ausrückrippenteile 50 und 51 sind in diesem Ausführungsbeispiel insbesondere konstruktiv identisch mit den Sperrrippenteilen 21 und 22.

Diese zwei Ausrückrippenteile 50, 51 erstrecken sich insofern jeweils ausgehend von einem Drücker- bzw. Anzeigekopfteile 52 des Auslöseelements 13 radial runterwärts 53 (siehe Figur 4) auf die Längsachse 6 der Stechhilfevorrichtung 1 zu, so dass diese von dem axial verschieblich gelagerten Spannschlittenteil 27 axial gut zugänglich an dem Auslöseelement 13 angeordnet sind.

Insbesondere durchstoßen die zwei Ausrückrippenteile 50, 51 eine Längserstreckungsschiebeebene 54 (siehe Figur 3) des axial verschieblich gelagerten Spannschlittenteils 27, wenn sich das Auslöseelement 13 noch nicht in der ausgerückten Anzeigeposition 43 befindet.

Diese Längserstreckungsschiebeebene 54 befindet sich zwischen der Mittellängsachse der Stechhilfevorrichtung 1 und dem Auslöseelement 13 bzw. dem Drücker- und Anzeigekopfteil 52.

Um mit dem axial verschieblich gelagerten Spannschlittenteil 27 insbesondere verkantungsfrei wechselwirken zu können, weisen die beiden Ausrückrippenteile 50 und 51 jeweils eine in Bezug auf die Stechrichtung 18 geneigte Ausrückschräge 55 und 56 auf, die dementsprechend ebenfalls gegenüber der Längserstreckungsschiebeebene 54 geneigt angeordnet sind.

Ein Wechselwirken zwischen dem axial verschieblich gelagerten Spannschlittenteil 27 und dem Auslöseelement 13 ist weiter verbessert, da das axial verschieblich gelagerte Spannschlittenteil 27 mit zwei Betätigungselementen 57 und 58 zum Ausrücken des Auslöseelements 13 ausgestattet ist.

Diese Betätigungselemente 57, 58 erstrecken sich in Stechrichtung 18 gesehen an der Kopfseite 59 des axial verschieblich gelagerten Spannschlittenteils 27 in Gestalt von zwei parallel zueinander verlaufenden Armteilen 60 und 61.

Diese Armteile 60, 61 stellen wiederum jeweils eine in Bezug auf die Stechrichtung 18 geneigte Betätigungsschräge 62 und 63 (siehe Figur 2) zur Verfügung, welche als Gegenflächen mit den geneigten Ausrückschrägen 55 und 56 zusammenwirken, wenn das axial verschieblich gelagerte Spannschlittenteil 27 axial in Stechrichtung 18 bewegt wird, und hierbei das Auslöseelement 13 radial in die ausgerückte Anzeigeposition 43 bewegt wird.

Die Stechhilfevorrichtung 1 verfügt noch über eine Halteeinrichtung 70 zum temporären Halten des Auslöseelements 13 in der ausgerückten Anzeigeposition 43.

In diesem Ausführungsbeispiel ist die Halteeinrichtung 70 derart ausgestaltet, dass sie am Auslöseelement 13 eine Vielzahl an Schnappelementen 71 (hier nur exemplarisch beziffert) und am Gehäuse 2 eine Vielzahl an von diesen Schnappelementen 71 hinterschnappbare Erhebungselemente 72 (nur exemplarisch beziffert) umfasst (siehe insbesondere Figur 6).

Während sich insbesondere das axial verschieblich gelagerte Spannschlittenteil 27 der Stechhilfevorrichtung 1 hinsichtlich der Darstellungen gemäß der Figuren 1 und 2 noch in der hinteren Ausgangsposition 29 befindet, ist dieses axial verschieblich gelagerte Spannschlittenteil 27 in der Darstellung gemäß der Figur 3 bereits in Stechrichtung 18 derart axial händisch nach vorne geschoben, dass dessen geneigte Betätigungsschrägen 62 und 63 an den geneigten Ausrückschrägen 55 und 56 des Auslöseelements 13 anliegen. Das Auslöseelement 13 ist hierbei radial noch nicht ausgerückt.

Übt ein Benutzer nun beabsichtigt oder unbeabsichtigt eine händische Betätigungskraft 74 auf das Auslöseelement 13 aus, wird das axial verschieblich gelagerte Spannschlittenteil 27 in Stechrichtung 18 blockiert. Insofern ist konstruktiv einfach die Sperreinrichtung 20 ohne zusätzliche Bauteilkomponenten realisiert. Auch eine zusätzliche Bedienung der Sperreinrichtung 20 erübrigt sich.

Wird das Auslöseelement 13 jedoch nicht gedrückt, kann das axial verschieblich gelagerte Spannschlittenteil 27 weiter in Stechrichtung 18 verschoben werden, wie in den Figuren 4 und 5 gezeigt.

Die Darstellungen gemäß der Figuren 4 und 5 zeigen das axial verschieblich gelagerte Spannschlittenteil 27 nun ein einer maximal in Stechrichtung 18 verlagerten Maximalschiebeposition 73, in welcher sich das Auslöseelement 13 bereits in der ausgerückten Anzeigeposition 43 befindet. In dieser vorderen Maximalschiebeposition 73 untergreifen die beiden Armteile 60 und 61 der Betätigungselemente 57 und 58 vollständig die beiden Ausrückrippenteile 50 und 51 des Auslöseelements 13 und heben dieses mit den Schnappelementen 71 der Halteeinrichtung 70 über die Erhebungselemente 72 der Halteeinrichtung 70, wodurch das Auslöseelement 13 in der ausgerückten Anzeigeposition 43 auch dann verbleibt, wenn das axial verschieblich gelagerte Spannschlittenteil 27 im nächsten Augenblick mithilfe von Federkräften entgegen der Stechrichtung 18 wieder in die hintere Ausgangsposition 29 zurückschnellt bzw. zurückgeschnellt ist (siehe Figur 6).

Die Darstellung gemäß der Figur 7 zeigt die Stechhilfevorrichtung 1 bereits in einem ausgelösten Zustand, bei welchem das Auslöseelement 13 durch eine händische Betätigungskraft 74 und durch Überwindung der von der Halteeinrichtung 70 aufgebrachten Haltekräfte radial in das Gehäuse 2 hinein gedrückt und die Lanzette 12 mit der Stechnadel 11 bereits in der Endstechlage 75 angeordnet ist.

Der vorstehend beschriebene Spannmechanismus kann auch angewendet werden, wenn das Spannschiebeelement 14 alternativ am Endbereich 7 als Druckknopf (hier nicht gezeigt) oder dergleichen platziert ist.

Vorteilhafterweise übernimmt das Auslöserelement 13 vorliegend neben der eigentlichen Auslösefunktion auch noch zwei weitere Funktionen, nämlich einerseits die einer Sperreinrichtung 20 und andererseits die einer Anzeigeeinrichtung 40.

Von enormen Vorteil ist hier, dass die Sperreinrichtung 20 und die Anzeigeeinrichtung 40 baugleich bzw. zumindest hinsichtlich ihrer wesentlichen Bauteilkomponenten baugleich ausgestaltet sind, so dass die Stechhilfevorrichtung 1 trotz dieser Funktionen konstruktiv einfach baut und zudem auch für einen ungeübten Benutzer einfach zu handhaben ist.

Es versteht sich, dass es sich bei dem vorstehend erläuterten Ausführungsbeispiel lediglich um eine erste Ausgestaltung der erfindungsgemäßen Stechhilfevorrichtung handelt. Insofern beschränkt sich die Ausgestaltung der Erfindung nicht auf dieses eine Ausführungsbeispiel.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Stechhilfevorrichtung
- 2: zweiteiliges Gehäuse
- 3: Oberschalenteil
- 4: Unterschalenteil
- 5: Längserstreckung
- 6: Längsachse
- 7: Endbereich
- 8: Kopfbereich
- 9: Schraubkappe
- 10: Durchführungsöffnung
- 11: Stechnadel
- 12: Lanzette
- 13: Auslösedrückelement
- 14: Spannschiebeelement
- 15: äußere Gehäuseoberfläche
- 16: Auswerferschiebeelement
- 17: Axialrichtungen
- 18: Stechrichtung
- 19: Radialrichtungen
- 20: Sperreinrichtung
- 21: linkes Sperrrippenteil
- 21A: linke Sperrschräge
- 22: rechtes Sperrrippenteil
- 22A: rechte Sperrschräge
- 23: linkes Anschlagteil
- 23A: linke Anschlagschräge
- 24: rechtes Anschlagteil
- 24A: rechte Anschlagschräge
- 25: Spanneinheit
- 27: axial verschieblich gelagertes Spannschlittenteil
- 27A: Hülsenteil des Spannschlittenteils
- 28: Spannschlittenfederelement
- 29: hintere Ausgangsposition
- 32: axial verlagerbares Lanzettenhalterelement
- 35: Hülsenteil einer Rasteinrichtung
- 38: Stehbolzen
- 39: Schraubenaufnahmelaschen
- 40: Anzeigeeinrichtung
- 41: stechbereiter Betriebszustand
- 42: nicht stechbereite Betriebszustände
- 43: ausgerückte Anzeigeposition
- 50: linkes Ausrückrippenteil
- 51: rechtes Ausrückrippenteil
- 52: Drücker- bzw. Anzeigekopfteil
- 53: runterwärts
- 54: Längserstreckungsschiebeebene
- 55: geneigte linke Ausrückschräge
- 56: geneigte rechte Ausrückschräge
- 57: rechtes Betätigungselement
- 58: linkes Betätigungselement
- 59: Kopfseite
- 60: rechtes Armteil
- 61: linkes Armteil
- 62: geneigte rechte Betätigungsschräge
- 63: geneigte linke Betätigungsschräge
- 70: Halteeinrichtung
- 71: Schnappelemente
- 72: Erhebungselemente
- 73: vordere Maximalschiebeposition
- 74: händische Betätigungskraft
- 75: Endstechlage

## Patentansprüche

1. Stechhilfevorrichtung (1) zur Blutprobenentnahme mit einem Gehäuse (2), mit einem axial verlagerbaren Lanzettenhalterelement (32) zum Haltern einer Lanzette (12), mit einem Stechfederelement zum Beschleunigen des axial verlagerbaren Lanzettenhalterelements (32) in Stechrichtung (18) und mit einem Auslöseelement (13) zum Einleiten eines Stechvorgangs, bei dem das axial verlagerbare Lanzettenhalterelement (32) mittels des gespannten Stechfederelements in Stechrichtung (18) beschleunigt wird,
**dadurch gekennzeichnet, dass**
die Stechhilfevorrichtung (1) eine durch das Auslöseelement (13) auslösbare Sperreinrichtung (20) zum Sperren eines Stechfederelementespannvorgangs aufweist.

2. Stechhilfevorrichtung (1) nach Anspruch 1,
**dadurch gekennzeichnet, dass**
das Auslöseelement (13) derart in dem Gehäuse (2) der Stechhilfevorrichtung (1) angeordnet ist, dass durch eine in Bezug auf die Stechrichtung (18) radial gerichtete Betätigungsbewegung des Auslöseelements (13) eine Verschiebung eines axial verschieblich gelagerten Spannschlittenteils (27) einer Spanneinheit (25) der Stechhilfevorrichtung (1) in eine vordere Maximalschiebeposition (73) hinein blockiert ist.

3. Stechhilfevorrichtung (1) nach Anspruch 1 oder 2,
**dadurch gekennzeichnet, dass**
das Auslöseelement (13) wenigstens ein Sperrrippenteil (21, 22) zum zumindest teilweise Sperren einer axialen Spannbewegung (21) des axial verschieblich gelagerten Spannschlittenelements (27) in Stechrichtung (18) umfasst.

4. Stechhilfevorrichtung (1) nach Anspruch 3,
**dadurch gekennzeichnet, dass**
das wenigstens eine Sperrrippenteil (21, 22) sich von einem Drücker- bzw. Anzeigekopfteil (52) des Auslöseelements (13) ausgehend bis in eine Längserstreckungsschiebeebene (54) eines axial verschieblich gelagerten Spannschlittenteils (27) hinein erstreckt, in welcher das axial verschieblich gelagerte Spannschlittenteil (27) zumindest teilweise axial verschieblich angeordnet ist.

5. Stechhilfevorrichtung (1) nach Anspruch 4,
**dadurch gekennzeichnet, dass**
die Längserstreckungsschiebeebene (54) zwischen der Mittellängsachse (6) und dem Auslöseelement (13) angeordnet ist.

6. Stechhilfevorrichtung (1) nach einem der Ansprüche 1 bis 5,
**dadurch gekennzeichnet, dass**
das wenigstens eine Sperrrippenteil (21, 22) eine in Bezug auf die Stechrichtung (18) geneigte Sperrschräge (21 A, 22A) aufweist.

7. Stechhilfevorrichtung (1) nach einem der Ansprüche 1 bis 6,
**dadurch gekennzeichnet, dass**
die Sperreinrichtung (20) wenigstens ein zur Stechrichtung (18) fluchtendes Anschlagteil (23, 24) zum Anschlagen an das wenigstens eine Sperrrippenteil (21, 22) umfasst, wobei das Anschlagteil (23, 24) an einem axial verschieblich gelagerten Spannschlittenteil (27) angeordnet ist.

8. Stechhilfevorrichtung (1) nach Anspruch 7,
**dadurch gekennzeichnet, dass**
das wenigstens eine Anschlagteil (23, 24) eine Anschlagschräge (23A, 24A) umfasst, welche einer Sperrschräge (21 A, 22A) eines Sperrrippenteils (21, 22) des Auslöseelements (13) zugewandt und gegenüberliegend angeordnet ist.

## Claims

1. Lancing device (1) for taking blood samples, comprising a housing (2), an axially displaceable lancet holder element (32) for holding a lancet (12), a lancing spring element for accelerating the axially displaceable lancet holder element (32) in a lancing direction (18), and a release element (13) for initiating a lancing procedure, in which the axially displaceable lancet holder element (32) is accelerated in a lancing direction (18) by means of the tensioned lancing spring element, **characterised in that** the lancing device (1) comprises a blocking apparatus (20), which can be released by the release element (13), for blocking a lancing spring element tensioning action.

2. Lancing device (1) according to claim 1, **characterised in that** the release element (13) is arranged in the housing (2) of the lancing device (1) such that a displacement of an axially displaceably mounted tensioning carriage part (27) of a tensioning unit (25) of the lancing device (1) into a front maximum sliding position (73) is blocked by a radially directed actuating movement of the release element (13) relative to the lancing direction (18).

3. Lancing device (1) according to claim 1 or claim 2, **characterised in that** the release element (13) comprises at least one blocking rib part (21, 22) for blocking an axial tensioning movement (21) of the axially displaceably mounted tensioning carriage part (27) in a lancing direction (18) at least in part.

4. Lancing device (1) according to claim 3, **characterised in that** the at least one blocking rib part (21, 22) extends from a push-piece- and respectively display head part (52) of the release element (13) into a longitudinal-extension sliding plane (54) of an axially displaceably mounted tensioning carriage part (27), in which plane the axially displaceably mounted tensioning carriage part (27) is arranged axially displaceably at least in part.

5. Lancing device (1) according to claim 4, **characterised in that** the longitudinal-extension sliding plane (54) is arranged between the central longitudinal axis (6) and the release element (13).

6. Lancing device (1) according to any of claims 1 to 5, **characterised in that** the at least one blocking rib part (21, 22) comprises a blocking bevel (21 A, 22A) which is inclined relative to the lancing direction (18).

7. Lancing device (1) according to any of claims 1 to 6, **characterised in that** the blocking apparatus (20) comprises at least one stop part (23, 24), which is aligned with the lancing direction (18), for abutting the at least one blocking rib part (21, 22), the stop part (23, 24) being arranged on an axially displaceably mounted tensioning carriage part (27).

8. Lancing device (1) according to claim 7, **characterised in that** the at least one stop part (23, 24) comprises a stop bevel (23A, 24A) which faces a blocking bevel (21 A, 22A) of a blocking rib part (21, 22) of the release element (13) and is arranged opposite said blocking bevel.

## Revendications

1. Dispositif autopiqueur (1) pour le prélèvement d'un échantillon de sang, comportant un boîter (2), un porte-lancette (32) mobile axialement, destiné à maintenir une lancette (12), un élément ressort de piqûre pour accélérer le porte-lancette (32), mobile axialement, dans la direction de piqûre (18), et un élément déclencheur (13) destiné à déclencher un processus de piqûre, le porte-lancette (32) mobile axialement étant accéléré dans la direction de piqûre (18) au moyen de l'élément ressort de piqûre mis sous tension,
**caractérisé en ce que** le dispositif autopiqueur (1) comporte un dispositif d'arrêt (20), pouvant être déclenché par l'élément déclencheur (13) et destiné à bloquer le processus de mise sous tension del' élément ressort de piqûre.

2. Dispositif autopiqueur (1) selon la revendication 1, **caractérisé en ce que** l'élément déclencheur (13) est disposé dans le corps (2) du dispositif autopiqueur (1) de telle sorte que par un mouvement d'actionnement, dirigé radialement par rapport à la direction de piqûre (18), de l'élément déclencheur (13), un coulissement d'un élément formant chariot de tension (27), monté mobile axialement, d'une unité de mise sous tension (25) du dispositif autopiqueur (1) est bloqué dans une position de coulissement maximum (73) en avant.

3. Dispositif autopiqueur (1) selon les revendications 1 ou 2, **caractérisé en ce que** l'élément déclencheur (13) comporte au moins une partie à nervures d'arrêt (21, 22) pour bloquer au moins partiellement un mouvement de mise sous tension (21) axial de l'élément formant chariot de tension (27), mobile axialement, dans la direction de piqûre (18).

4. Dispositif autopiqueur (1) selon la revendication 3, **caractérisé en ce qu'**au moins une partie à nervures d'arrêt (21, 22) s'étend depuis une tête poussoir ou respectivement tête d'affichage (52) de l'élément déclencheur (13) jusque dans un plan de coulissement longitudinal (54) d'un élément formant chariot de tension (27) monté mobile axialement, dans lequel l'élément formant chariot de tension (27) monté mobile axialement est disposé au moins en partie de manière à coulisser axialement.

5. Dispositif autopiqueur (1) selon la revendication 4, **caractérisé en ce que** le plan de coulissement longitudinal (54) est disposé entre l'axe longitudinal médian (6) et l'élément déclencheur (13).

6. Dispositif autopiqueur (1) selon l'une quelconque des revendications 1 à 5, **caractérisé en ce que** ladite au moins une partie à nervures d'arrêt (21, 22) comporte une rampe de blocage (21 A, 22A) inclinée par rapport à la direction de piqûre (18).

7. Dispositif autopiqueur (1) selon l'une quelconque des revendications 1 à 6, **caractérisé en ce que** le dispositif d'arrêt (20) comporte au moins un élément de butée (23, 24), aligné dans la direction de piqûre (18) et destiné à venir buter contre ladite au moins une partie à nervures d'arrêt (21, 22), ledit élément de butée (23, 24) étant disposé sur un élément formant chariot de tension (27) monté mobile axialement.

8. Dispositif autopiqueur (1) selon la revendication 7, **caractérisé en ce que** ledit au moins un élément de butée (23, 24) comporte une rampe de butée (23A, 24A) qui est disposée en face d'une rampe de blocage (21 A, 22A) d'une partie à nervures d'arrêt (21, 22) de l'élément déclencheur (13) et est orientée vers ladite partie à nervures d'arrêt.
